# FASCICULE DE BREVET EUROPEEN

(11) **EP 1 704 136 B1**
(45) Date de publication et mention de la délivrance du brevet: **13.08.2008**
(21) Numéro de dépôt: 05717370.0
(22) Date de dépôt: 06.01.2005
(51) Int. Cl.: C07C 50/32, C07C 321/28, C07C 321/16, A61K 31/122, A61K 31/10, A61P 35/00

(54) **DERIVES DU GOSSYPOL, LEUR METHODE D OBTENTION ET LEURS UTILISATIONS**
GOSSYPOLDERIVATE, VERFAHREN ZU DEREN HERSTELLUNG UND DEREN ANWENDUNGEN
GOSSYPOL DERIVATIVES, PRODUCTION METHOD THEREOF AND USES OF SAME

(30) Priorité: 08.01.2004 FR 0400125
(43) Date de publication de la demande: 27.09.2006
(73) Titulaire: CENTRE NATIONAL DE LA RECHERCHE SCIENTIFIQUE (CNRS), 75794 Paris Cedex 16 (FR); INSTITUT NATIONAL DE LA SANTE ET DE LA RECHERCHE MEDICALE (INSERM), 75654 Paris Cédex 13 (FR)
(72) Inventeur: DAO, Vi-Thuy, F-92160 Antony (FR); DE GUNZBURG, Jean, F-75016 Paris (FR); MICHELOT, Robert, F-92160 Antony (FR); DE MIL, Christophe, Olivier, F-75014 Paris (FR)
(74) Mandataire: Grosset-Fournier, Chantal Catherine
(86) Numéro de dépôt international: PCT/FR2005/000022
(87) Numéro de publication internationale: WO 2005/073158

(56) Documents cités:
- WO-A-01/00554
- US-A- 5 385 936
- V-T. DAO ET AL: "New thioderivatives of gossypol and gossypolone, as prodrugs of cytotoxic agents" BIOORGANIC AND MEDICINAL CHEMISTRY, vol. 11, 2003, pages 2001-2006, XP002294825 cité dans la demande
- DAO V-T ET AL: "Synthesis and cytotoxicity of gossypol related compounds" EUROPEAN JOURNAL OF MEDICINAL CHEMISTRY, EDITIONS SCIENTIFIQUE ELSEVIER, PARIS, FR, vol. 35, no. 9, septembre 2000 (2000-09), pages 805-813, XP004331214 ISSN: 0223-5234

## Description

La présente invention a pour objet des dérivés du gossypol, et plus particulièrement des dérivés réduits et oxydés de la gossypolone, ainsi que leurs procédés d'obtention, et leurs utilisations, notamment dans des compositions pharmaceutiques destinées au traitement des cancers.

Le gossypol est généralement extrait des graines de cotonnier du genre *Gossypium* (famille des malvacées). La teneur varie de 0,1 à 0,64% selon la variété de *Gossypium* considérée.

La première extraction du gossypol fut réalisée grossièrement par Kuhlmann en 1861, il appela la substance « bleu des grains de coton ». En 1866, Longmore constata que l'huile des graines du coton contient un composé coloré, isolé sous forme d'un solide brun et qui est indiscutablement du gossypol impur. Ce n'est qu'en 1899 que Marchlewski obtint la forme cristalline pure, extraite des graines de coton, qu'il appela gossypol (Adams et al. (1960) Chem. Rev. 60:555-574).

S'inspirant des travaux de leurs prédécesseurs, Withers et Carruth, en 1915, améliorèrent d'une manière significative l'extraction du gossypol et leur méthode reste encore de nos jours un des meilleurs procédés utilisés (Adams et al. (1960) Chem. Rev. 60:555-574).

La molécule du gossypol se présente sous forme de deux sous-unités naphtaléniques symétriques, qui sont substituées chacune par trois fonctions phénoliques et une fonction aldéhyde.

Elle existe sous trois formes tautomères représentées par les formules suivantes : formes aldéhydique, hemiacétalique et quinone-méthinique. Sa masse molaire est de : 518.5 g/mol (C₃₀H₃₀O₈).

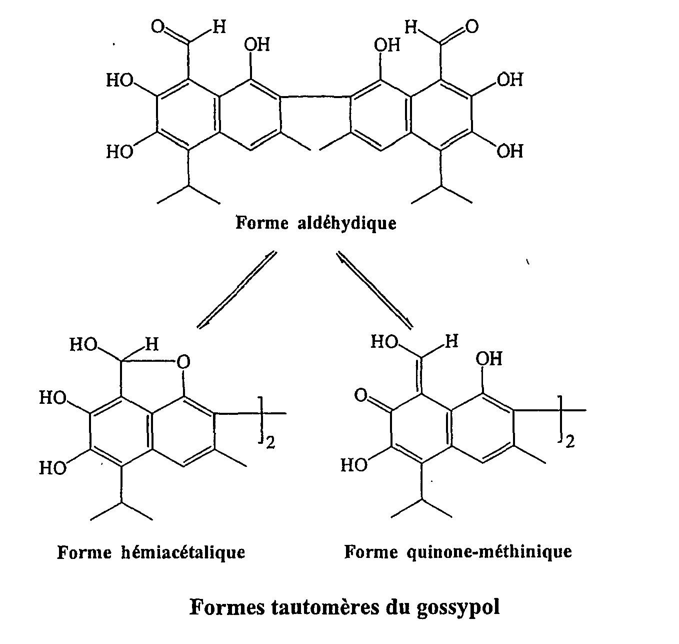

L'analyse du pouvoir rotatoire du gossypol, a révélé la présence de deux isomères. Le (+) gossypol est principalement abondant dans l'écorce du *Thephesia populnea* et certaines variétés de cotonniers (*Gossypium hirsutum*), tandis que l'isomère lévogyre (le (-) gossypol) est prépondérant dans d'autres variétés de cotonniers telles que le *Gossypium barbadense* (Zhou & Lin (1988) Contraception 37:239-245; Cass et al. (1991) Phytochemistry 30:2655-2657).

La méthode utilisée pour la mesure de l'excès énantiomérique dans les graines des différents cotonniers est celle que Matlin et Zhou ont appliquée à partir de bases de Schiff chirales du gossypol (+) ou (-) en effectuant leur séparation par HPLC (Zhou & Lin (1988) Contraception 37:239-24.5 ; Matlin et al. (1984) Journal of High Resolution Chromatography and Chromatography Communication 7:629-631 ; Fish et al. (1995) Tetrahedron asymetry 6:873-876).

La gossypolone est obtenue par oxydation du gossypol. A ce titre la méthode d'obtention décrite par Hass & Shirley (1965) J. Org. Chem. 30:4111-4113, utilisant le chlorure ferrique est la plus courante.

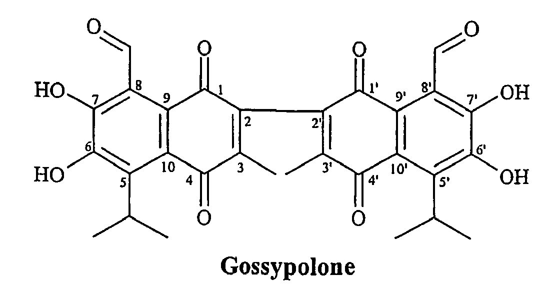

Le gossypol possède de nombreuses activités biologiques, qui sont dans une certaine mesure partagées par ses dérivés. S'il fût d'abord étudié principalement pour ses propriétés contraceptives ou antiparasitaires (contre les virus ou les protozoaires), ce sont actuellement ses activités antitumorales qui intéressent les médecins. Comme sa toxicité à doses élevées n'est pas négligeable et entraîne de multiples effets secondaires chez l'Homme, la recherche s'est orientée vers des dérivés ciblant plus spécifiquement les cellules cancéreuses.

Les nombreuses données bibliographiques sur les activités des dérivés du gossypol indiquent que l'activité est liée à la présence sur le système binaphtalénique des groupements phénoliques libres en 6,6' et 7,7'. En effet, le blocage de ces fonctions phénol, dans les dérivés méthoxyethers, abolit toute toxicité. La toxicité du gossypol est aussi associée aux fonctions aldéhydes ou à la présence d'une fonction électrophile comme une ènamine dans la même position. On imagine facilement que ce centre électrophile peut se lier avec nombre d'entités nucléophiles jouant un rôle important dans le milieu biologique. Par exemple, les groupements amines de la lysine des protéines ou des peptides se couplent facilement avec le gossypol.

A ce titre, l'article Dao et al. (2003) Bioorg. Med. Chem. 11:2001-2006 décrit des dérivés dithiolane et dithiane du gossypol et de la gossypolone, possédant une cytotoxicité inférieure à celle du gossypol et de la gossypolone sur la lignée cellulaire KB. Ces dérivés sont, de plus, susceptibles d'être modifiés au contact d'ions nitronium, qui sont préférentiellement présents au niveau de certaines tumeurs, en des composés fortement cytotoxiques. Ces composés n'agissent donc pas directement.

La présente invention a pour but de fournir de nouveaux dérivés de la gossypolone présentant une cytotoxicité inférieure ou égale à celle du gossypol et de la gossypolone, induisant moins d'effets secondaires que le gossypol et la gossypolone, et pouvant inhiber directement des protéines kinases susceptibles d'être impliquées dans le développement et le maintien des tumeurs.

L'invention a également pour but de fournir de nouveaux procédés de synthèse des dérivés susmentionnés.

L'invention a aussi pour but de fournir de nouvelles compositions pharmaceutiques, comprenant les dérivés de l'invention, et d'utiliser lesdites compositions dans le cadre du traitement des cancers, ces compositions présentant l'avantage de ne pas être toxiques aux doses utilisées pour l'organisme, et d'inhiber spécifiquement certaines protéines kinases susceptibles d'être impliquées dans la croissance des cellules cancéreuses.

La présente invention concerne des composés de formule générale (1) : 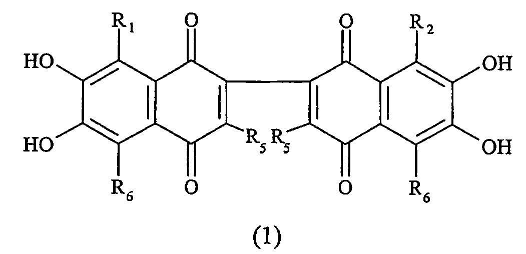
dans laquelle, indépendamment l'un de l'autre, R₁ et R₂ représentent :
   -OH, ou
   -CH₂-O-R₃, ou
   -CH₂-S-R₃, ou 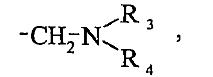
      R₃ et R₄ représentent, indépendamment l'un de l'autre, -H ou un groupement carboné de 1 à 10 atomes de carbones, saturé ou non, comprenant éventuellement substitué un ou plusieurs groupements hétéroatomiques,
      R₅ représente un groupement méthyle,
      et R₆ représente un groupement isopropyle.

On désigne par « groupement hétéroatomique » un groupement comprenant au moins un hétéroatome, tel que O, N, S, P, Cl, Br, F ou I, ledit groupement pouvant par exemple correspondre à -OH, -NH₂, -SH.

Selon un mode de réalisation particulier, indépendamment les uns des autres, R₃ et R₄ représentent:
-H, un groupement alkyle, un groupement hétéroalkyle, tel qu'un thioalkyle, un hydroxyalkyle, un aminoalkyle, un halogénoalkyle ou un alkoxyalkyle, un groupement cycloalkyle, un groupement alcényle, un groupement hétéroalcényle, un groupement aryle, un groupement hétéroaryle, tel qu'un thioaryle, un hydroxyaryle, un aminoaryle, un halogénoaryle ou un alkoxyaryle, ou un groupement arylalkyle.

Selon un mode de réalisation préféré R₃, et comprennent, indépendemment l'un de l'autre, de 1 à 8 atomes de carbone.

Selon un autre mode de réalisation particulier, R₁ et R₂ sont identiques.

Selon encore un autre mode de réalisation particulier, R₃ représente -H ou un groupement alkyle ou hydroxyalkyle de 1 à 4 atomes de carbones, ou un groupement benzyle, le cas échéant substitué par un ou plusieurs groupements alkyles ou alkoxy de 1 à 4 atomes de carbone, et/ou par un ou plusieurs atomes d'halogène.

Selon un mode de réalisation préféré l'invention concerne des composés tels que définis ci-dessus, de formule générale (1bis) : 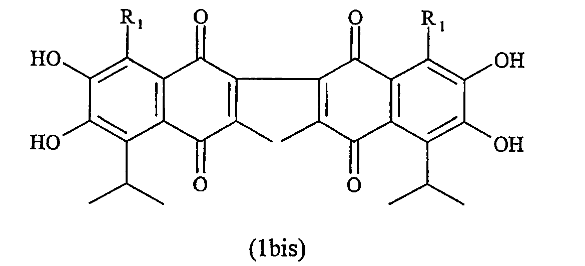 dans laquelle R₁ représente :
-OH, ou
-CH₂-O-R₃, ou
-CH₂-S-R₃,
R₃ étant tel que défini ci-dessus.

L'invention concerne en particulier des composés tels que définis ci-dessus, de formule générale (2) : 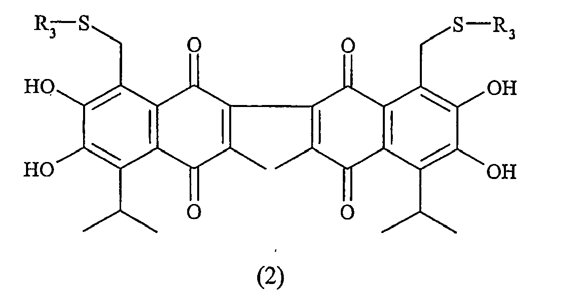 dans laquelle R₃ est tel que défini ci-dessus.

L'invention concerne notamment des composés tels que définis ci-dessus, de formule générale (3) : 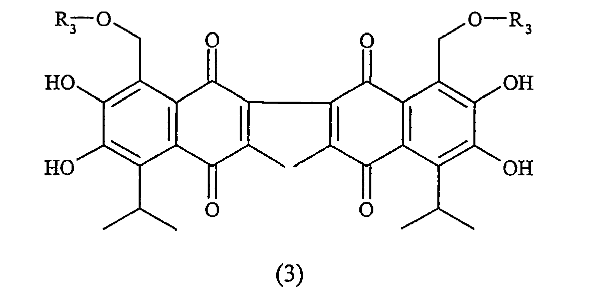 dans laquelle R₃ est tel que défini ci-dessus.

L'invention concerne plus particulièrement des composés tels que définis ci-dessus, de formule suivante :

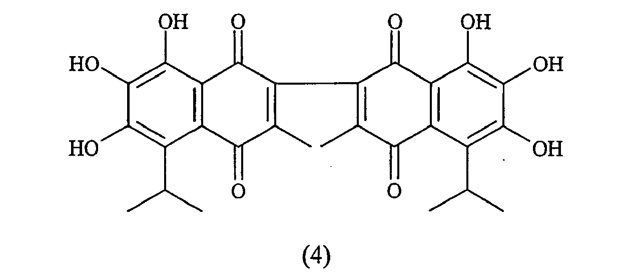

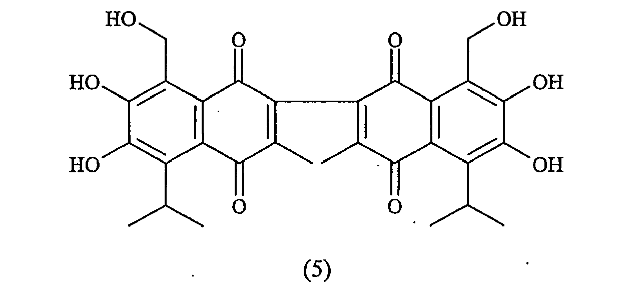

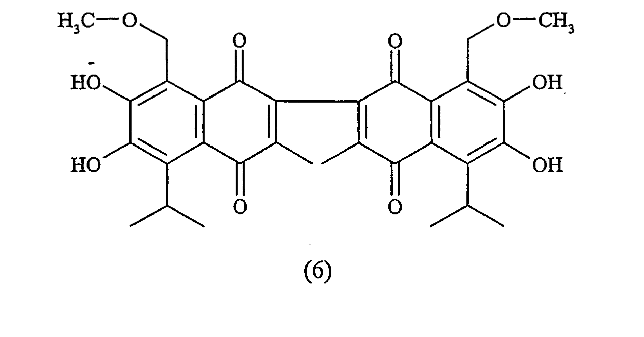

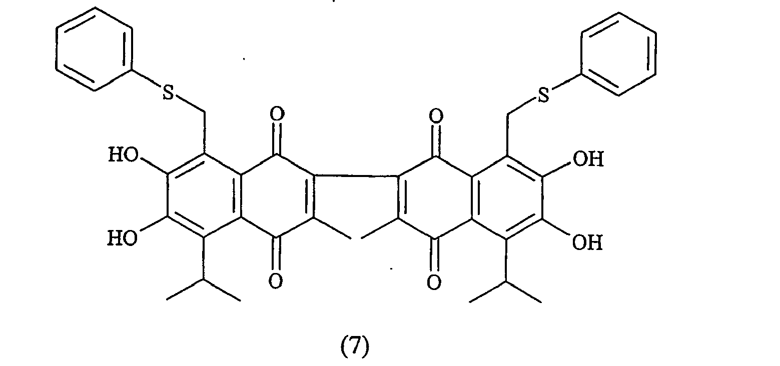

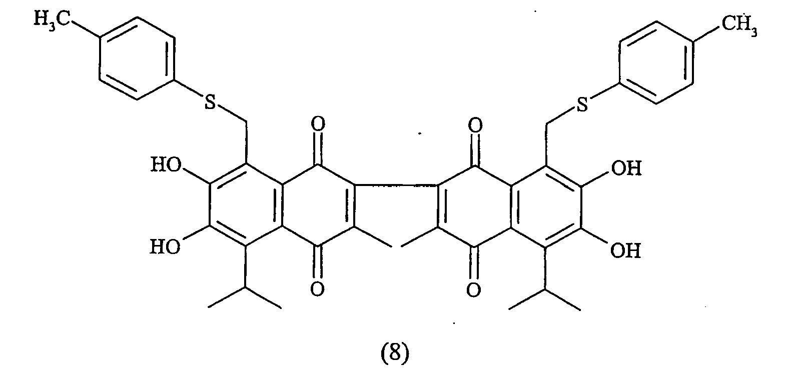

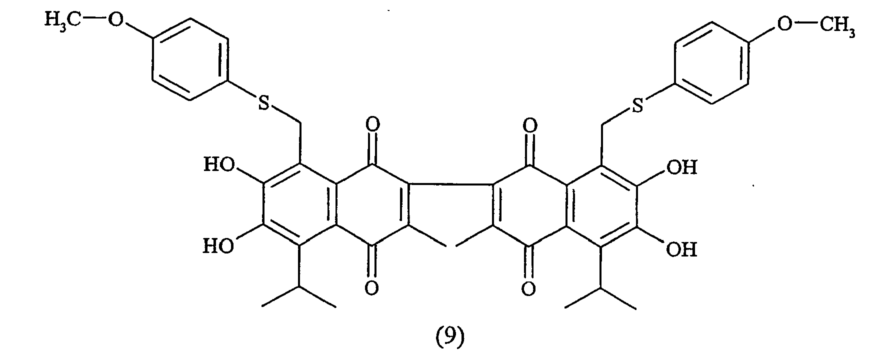

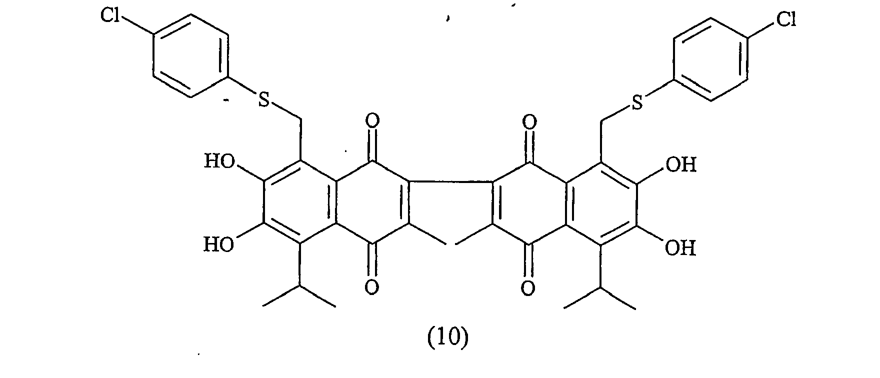

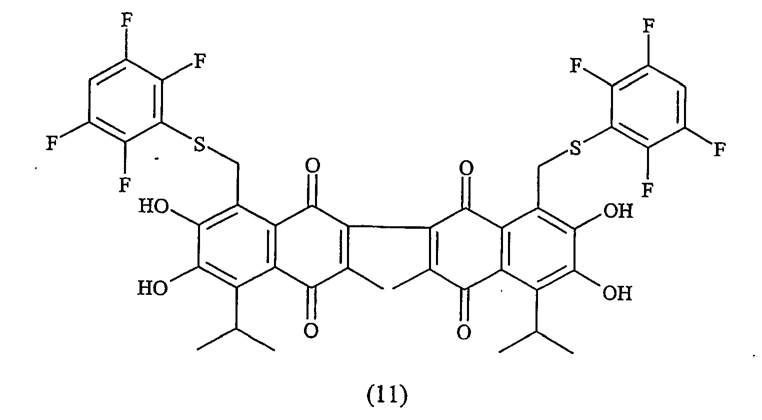

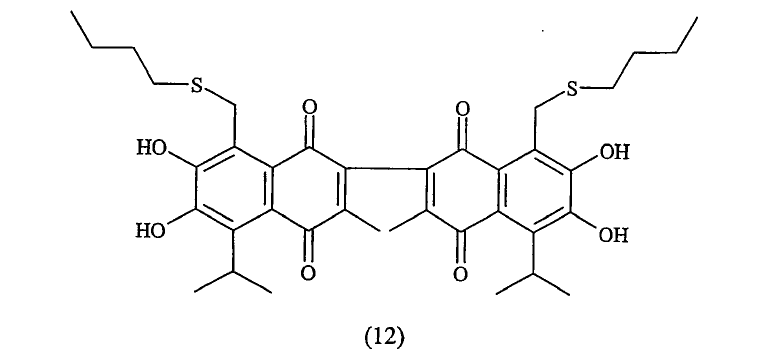

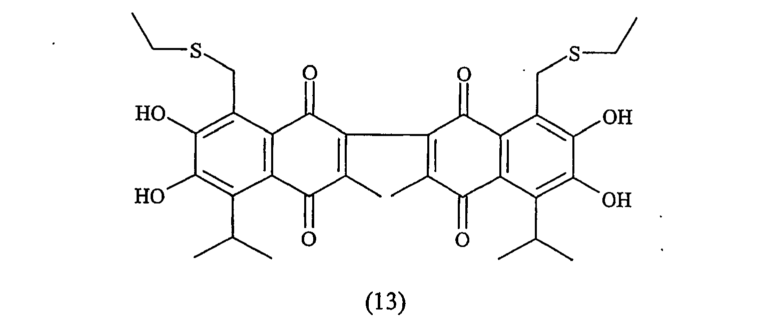

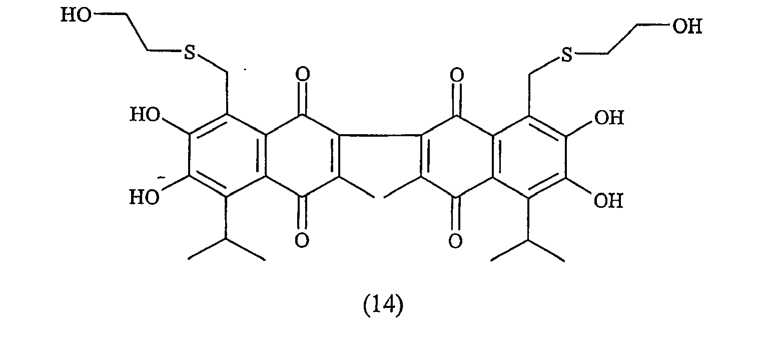

La présente invention concerne également une composition pharmaceutique comprenant à titre de substance active au moins un composé tel que défini ci-dessus, ou leurs sels pharmaceutiquement acceptables.

Une telle composition est avantageuse car elle vise à inhiber des protéines kinases susceptibles d'être impliquées dans le développement et/ou le maintien des tumeurs, et induit moins d'effets secondaires que les compositions pharmaceutiques contenant du gossypol et/ou de la gossypolone.

Selon un mode de réalisation particulier la composition pharmaceutique comprend à titre de substance active le composé (4) et/ou le composé (14), tels que définis ci-dessus.

La présente invention concerne également l'utilisation d'un composé tel que défini ci-dessus, pour la préparation d'un médicament destiné au traitement des cancers, des maladies parasitaires, telles que le paludisme, des viroses ou des rejets de greffe, ou à la contraception masculine.

Les maladies parasitaires comprennent notamment les maladies dont l'agent étiologique est un protozoaire, tel que *Plasmodium falciparum* par exemple.

L'utilisation des composés de l'invention est avantageuse car ils sont susceptibles de remplir des fonctions thérapeutiques similaires à celles du gossypol et de la gossypolone tout en induisant moins d'effets secondaires que le gossypol et la gossypolone.

Selon un mode de réalisation préféré, le composé (4) et/ou le composé (14) tel que définis ci-dessus, sont utilisés pour la préparation d'un médicament destiné au traitement des cancers, tels que les mélanomes, les cancers du colon, les cancers du poumon, les glioblastomes, les adénocarcinomes, les cancers de la prostate ou les cancers du sein.

L'utilisation des composés (4) et (14) est avantageuse car ces composés inhibent spécifiquement et directement l'activité des kinases SGK et PRAK, susceptibles d'être impliquées dans le développement et/ou le maintien des tumeurs, et induisent moins d'effets secondaires que le gossypol et la gossypolone.

La présente invention concerne également un procédé de préparation d'un composé de formule générale (2) tel que défini ci-dessus ou d'un composé de formule (3) tel que défini ci-dessus, caractérisé en ce qu'il comprend :
- une étape de réduction de la gossypolone pour former le composé de formule (5) tel que défini ci-dessus, et
- une étape de substitution dudit composé de formule (5) par un composé de formule R₃-SH pour former un composé de formule (2), ou par un composé de formule R₃-OH pour former un composé de formule (3).

La présente invention concerne également un procédé de préparation d'un composé de formule générale (4), tel que défini ci-dessus, caractérisé en ce qu'il comprend une étape d'oxydation de la gossypolone.

### EXEMPLES

### Exemple 1

### Synthèse d'un dérivé oxydé de la gossypolone, la 6,7,8,6',7',8'-hexahydroxy-5,5'-diisopropyl-3,3'-diméthyl-[2,2']binaphtalényl-1,4,1',4'-tétraone (4)

1g de gossypolone (1,83 mmole) est dissous dans 50ml d'acétone et mis sous argon, on ajoute 8ml de H₂O₂ 30% en 0,5 ml chaque fois, on laisse tourner à température ambiante. Après 24h, la réaction, suivie par HPLC, est terminée. Le mélange réactionnel est lavé avec une solution concentrée de FeSO₄, puis avec NaCl saturé, séché sur Na₂SO₄, et évaporé. Le produit est obtenu par une précipitation dans l'éther et l'hexane.
Rendement : 95%
Spectrométrie de masse (ESI) : 521 (M-H)
IC50 (lignée cellulaire KB) = 10⁻⁶ M.
La cytotoxicité du composé vis-à-vis de la lignée cellulaire tumorale KB a été mesurée comme cela est décrit dans *Dao et al.* (2003) *Bioorg. Med. Chem.* **11**:2001-2006.

Les données de RMN ¹H (à gauche) et ¹³C (à droite) sont portées sur la formule (4) :

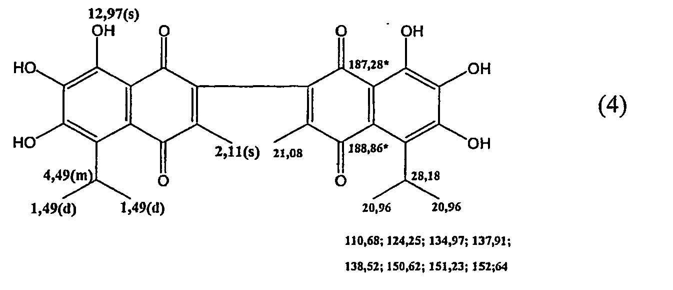

### Exemple 2

### Synthèse d'un dérivé réduit de la gossypolone, la 6,7,6',7'-tétrahydroxy-8,8'-bis-hydroxyméthyl-5,5'-düsopropyl-3,3'-diméthyl-[2,2']binaphtalényl-1,4,1',4'-tétraone (5)

1g de la gossypolone (1,83 mmole) et 4.eqv mol de NaBH₃CN sont ajoutés dans 50 ml de méthanol et mis sous argon. Ensuite, une solution de HCl-MeOH 3N (450µl) est ajoutée goutte à goutte pendant 5 min à 25°C. Après 15 min, la réaction, suivie par HPLC, est terminée. Le mélange réactionnel est versé dans un ballon qui contient 300 ml HCl 1N, un précipité orange se forme. On sépare le résidu et le dissout dans l'éther, la phase éthérée est lavée avec de l'eau (3 fois), ensuite, avec NaCl saturé, séché sur Na₂SO₄, et évaporé. Le produit est obtenu par une précipitation dans l'éther et l'hexane. Rendement : 95%
Spectrométrie de masse (ESI) : 573 (M+Na)
IC50 (lignée cellulaire KB) = 10⁻⁶ M

Les données de RMN ¹H (à gauche) et ¹³C (à droite) sont portées sur la formule (5) :

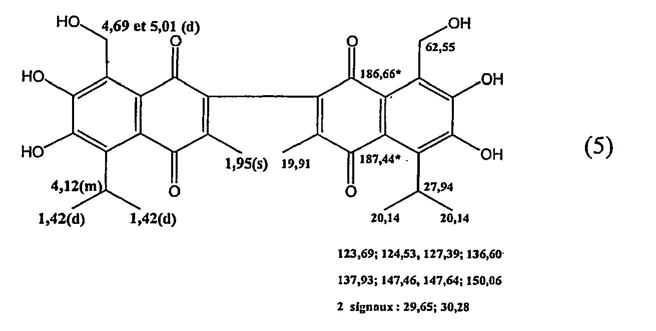

### Exemple 3

### Synthèse d'un dérivé thioéther du composé de formule (2), la 6,7,6',7'-tétrahydroxy-5,5'-diisopropyl-3,3'-diméthyl-8,8'-bis-phénylsulfanylméthyl-[2,2']binaphtalényl-1,4,1',4'-tétraone (7)

A une solution de 200 mg du composé de formule (5) (0,36 mmole) dans 10 ml de THF à 0°C est ajouté 3.eqv mol d'anhydride trifluoroacetique (TFAA). Le mélange est laissé tourner à 0°C pendant 15 min. Ensuite on évapore pour éliminer le THF. Le mélange réactionnel est repris dans 10 ml d'éther et 3.eqv mol d'un monothiol, ici le benzènethiol, est ajouté. Après 24h, le mélange réactionnel est lavé avec NaHCO₃ 5% (3 fois) et avec de l'eau (3 fois), ensuite, avec NaCl saturé, séché sur Na₂SO₄, et évaporé. Le produit est obtenu par une précipitation dans l'éther et l'hexane. Rendement : 60-70%
Spectrométrie de masse (ESI) : 733 (M-H)
IC50 (lignée cellulaire KB) = 4x10⁻⁶ M

Les données de RMN ¹H (à gauche) et ¹³C (à droite) sont portées sur la formule (7) :

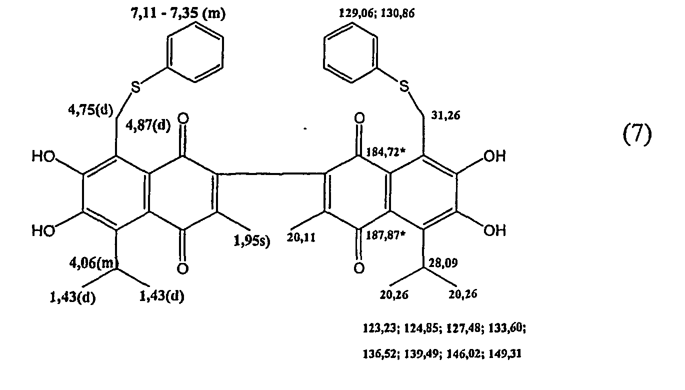

### Exemple 4

### Synthèse de la 6,7,6',7'-tétrahydroxy-5,5'-diisopropyl-3,3'-diméthyl-8,8'-bis-p-tolylsulfanylméthyl-[2,2']binaphtalényl-1,4,1',4'-tétraone (8)

La synthèse est réalisée essentiellement comme cela est décrit dans l'exemple 3, le monothiol utilisé étant le méthylbenzènethiol.
Rendement 60-70%
Spectrométrie de masse (ESI) : 761 (M-H)
IC50 (lignée cellulaire KB) = 6,5x10⁻⁶ M

Les données de RMN ¹H (à gauche) et ¹³C (à droite) sont portées sur la formule (8) :

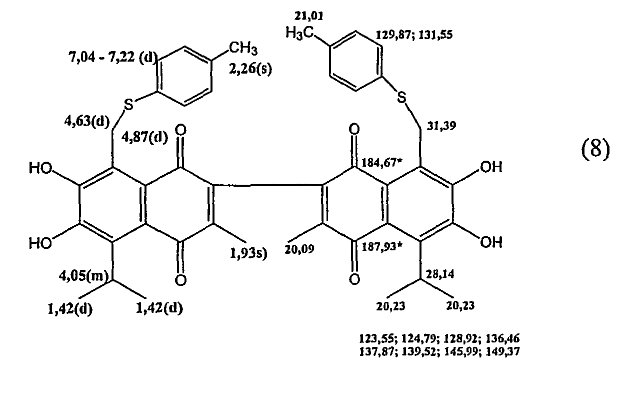

### Exemple 5

### Synthèse de la 6,7,6',7'-tétrahydroxy-5,5'-diisopropyl-8,8'-bis-(4-méthoxy-phénylsulfanylméthyl)-3,3'-diméthyl-[2,21]binaphtalényl-1,4,1',4'-tétraone (9)

La synthèse est réalisée essentiellement comme cela est décrit dans l'exemple 3, le monothiol utilisé étant le méthoxybenzènethiol.
Rendement 60-70%
Spectrométrie de masse (ESI) : 793 (M-H)
IC50 (lignée cellulaire KB) =6,5x10⁻⁶ M

Les données de RMN ¹H (à gauche) et ¹³C (à droite) sont portées sur la formule (9) :

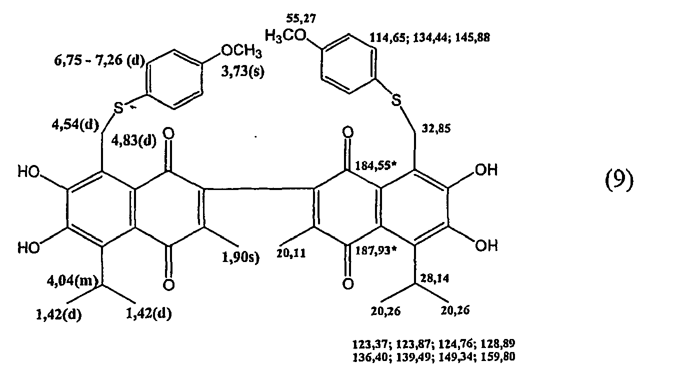

### Exemple 6

### Synthèse de la 6,7,6',7'-tétrahydroxy-5,5'-diisopropyl-8,8'-bis-(4-chloro-phénylsulfanylméthyl)-3,3'-diméthyl-[2,2']binaphtalényl-1,4,1',4'-tétraone (10)

Là synthèse est réalisée essentiellement comme cela est décrit dans l'exemple 3, le monothiol utilisé étant le para-chlorobenzènethiol.
Rendement 60-70%
Spectrométrie de masse (ESI) : 802 (M-H)
IC50 (lignée cellulaire KB) = 3x10⁻⁶ M

Les données de RMN ¹H (à gauche) et ¹³C (à droite) sont portées sur la formule (10) :

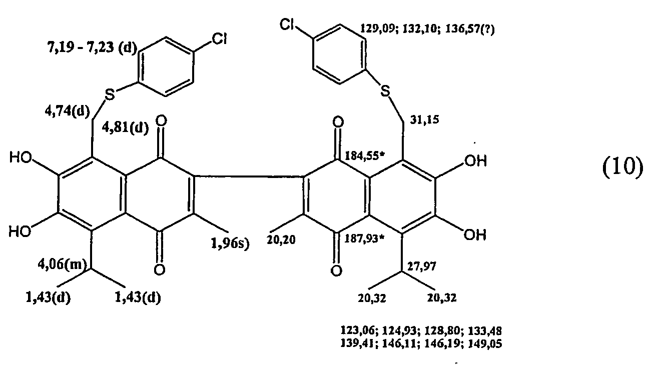

### Exemple 7

### Synthèse de la 6,7,6',7'-tétrahydroxy-5,5'-diisopropyl-8,8'-bis-(2,3,5,6-tétrafluoro-phénylsulfanylméthyl)-3,3'-diméthyl-[2,2']binaphtalényl-1,4,1',4'-tétraone (11)

La synthèse est réalisée essentiellement comme cela est décrit dans l'exemple 3, le monothiol utilisé étant le tétrafluorobenzènethiol.
Rendement 60-70%
Spectrométrie de masse (ESI) : 901 (M+Na)
IC50 (lignée cellulaire KB) = 8,5x10⁻⁶ M

Les données de RMN ¹H (à gauche) et ¹³C (à droite) sont portées sur la formule (11) :

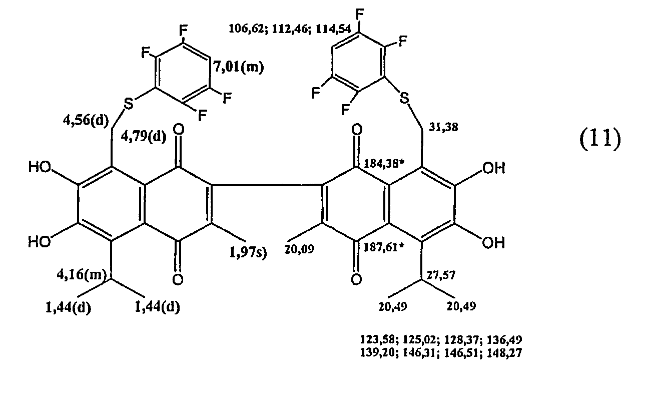

### Exemple 8

### Synthèse de la 8,8'-bis-butylsulfanylméthyl-6,7,6',7'-tétrahydroxy-5,5'-diisopropyl-3,3'-diméthyl-[2,2']binaphtalényl-1,4,1',4'-tétraone(12)

La synthèse est réalisée essentiellement comme cela est décrit dans l'exemple 3, le monothiol utilisé étant le thiobutane.
Rendement 60-70%
Spectrométrie de masse (ESI) : 693 (M-H)

Les données de RMN ¹H (à gauche) et ¹³C (à droite) sont portées sur la formule (12) :

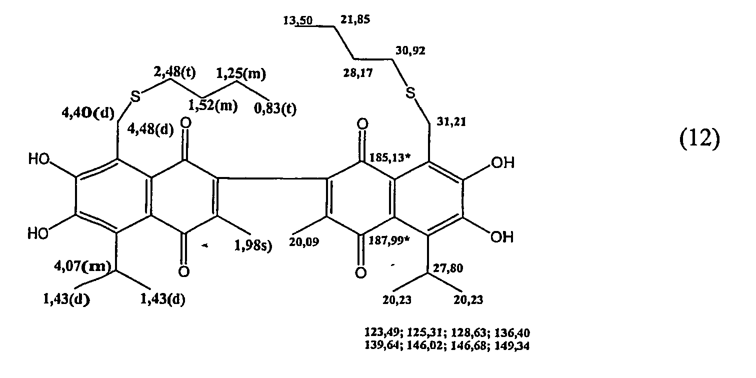

Alternativement, il est également possible d'utiliser du thioéthane à la place du thiobutane pour donner la 8,8'-bis-éthylsulfanylméthyl-6,7,6',7'-tétrahydroxy-5,5'-diisopropyl-3,3'-diméthyl -[2,2']binaphtalényl-1,4,1',4'-tétraone (13)

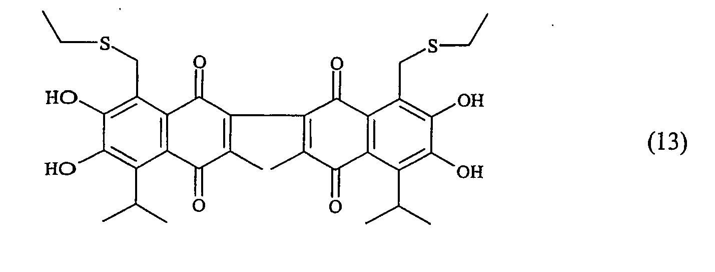

### Exemple 9

### Synthèse de la 6,7,6',7'-tétrahydroxy-8,8'-bis-(2-hydroxy-éthylsulfanylméthyl)-5,5'-diisopropyl-3,3'-diméthyl -[2,2']binaphtalényl-1,4,1',4'-tétraone (14)

La synthèse est réalisée essentiellement comme cela est décrit dans l'exemple 3, le monothiol utilisé étant le thioéthanol.
Rendement 60-70%
Spectrométrie de masse (ESI) : 669 (M-H)

Les données de RMN ¹H (à gauche) et ¹³C (à droite) sont portées sur la formule (14) :

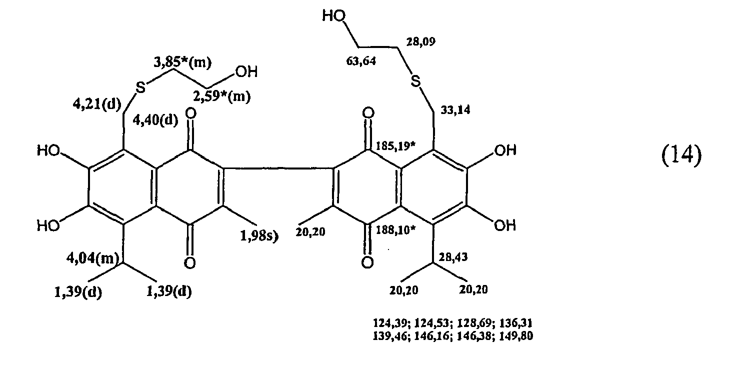

### Exemple 10

### Synthèse d'un dérivé méthyléther du composé de formule (2), la 6,7,6',7'-tétrahydroxy-8,8'-bis-méthoxyméthyl-5,5'-diisopropyl-3,3'-diméthyl-[2,2']binaphtalényl-1,4,1',4'-tétraone (6)

A une solution de 200 mg du composé de formule (5) (0,36 mmole) dans 10 ml de THF à 0°C est ajouté 3.eqv mol d'anhydride trifluoroacetique (TFAA). Le mélange est laissé tourner à 0°C pendant 15 min, ensuite on évapore pour éliminer le THF. Le mélange réactionnel est repris dans 10 ml d'éther et 3.eqv mol de méthylate (500 mg Na/ 10 ml MeOH) est ajouté. Après 5h, le mélange réactionnel est lavé avec de l'eau (3 fois), ensuite, avec NaCl saturé, séché sur Na₂SO₄, et évaporé. Le produit est obtenu par une précipitation dans l'éther et l'hexane.
Rendement : 70%.

Les données de RMN ¹H (à gauche) et ¹³C (à droite) sont portées sur la formule (6):

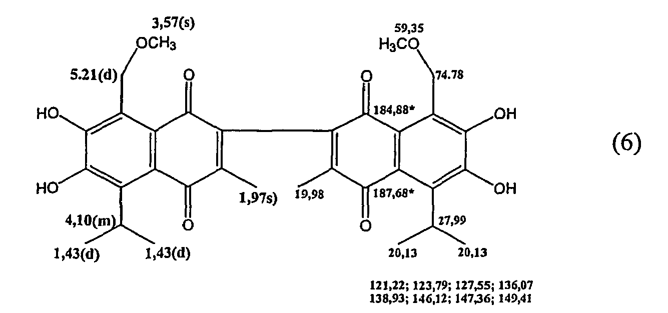

### Exemple 11

### Inhibition spécifique de l'activité de protéines kinases par le composé de formule (14)

L'action du composé de formule (14) a été testée sur une sélection de 29 protéine kinases différentes. L'activité de ces enzymes a été mesurée en présence de 10 µM du composé, selon la méthodologie générale décrite par Bain et al. (2003) Biochem. J. 371:199-204 et Davies et al. (2000) Biochem. J. 351:95-105.
Les résultats présentés dans le tableau 1 indiquent que le composé de formule (14) inhibe spécifiquement l'activité de deux enzymes, PRAK (kinase induite par les glucocorticoïdes régulée/activée par p38) et SGK (kinase induite par les glucocorticoïdes et le sérum), en diminuant l'activité de ces enzymes respectivement de 91% et de 93%.
La protéine SGK est connue pour être impliquée dans la régulation du cycle cellulaire et l'inhibition de l'apoptose (Buse et al. (1999) J. Biol. Chem. 274:7253-7263 ; Mikosz et al. (2001) J. Biol. Chem. 276: 16649-16654). Il s'agit donc d'une cible de choix dans le cadre de la lutte contre les cancers.

### Exemple 12

### Inhibition spécifique de l'activité de protéines kinases par le composé de formule (4)

L'action du composé de formule (4) a été testée sur une sélection de 29 protéine kinases différentes comme cela est décrit dans l'Exemple 11.
Les résultats présentés dans le tableau 1 indiquent que le composé de formule (4) inhibe également l'activité de deux enzymes, de manière spécifique, PRAK (kinase induite par les glucocorticoïdes régulée/activée par p38) et SGK (kinase induite par les glucocorticoïdes et le sérum), en diminuant l'activité de ces enzymes respectivement de 91 % et de 96%.

**Tableau 1 :**

| | Gossypol (10 µM) | | Gossypolone (10 µM) | | Composé (14) (10µM) | | Composé (4) (10µM) | |
|---|---|---|---|---|---|---|---|---|
| Protéine kinase | Activité résiduelle | Erreur standard | Activité résiduelle | Erreur standard | Activité résiduelle | Erreur standard | Activité résiduelle | Erreur standard |
| MKK1 | 86 | 5 | 76 | 10 | 90 | 15 | 59 | 3 |
| MAPK2/ERK2 | 65 | 1 | 46 | 9 | 58 | 5 | 52 | 9 |
| JNK/SAPK1c | 90 | 2 | 86 | 10 | 88 | 12 | 105 | 2 |
| SAPK2a/p38 | 99 | 2 | 108 | 1 | 102 | 9 | 105 | 8 |
| SAPK2b/p38β2 | 125 | 8 | 102 | 3 | 113 | 13 | 124 | 4 |
| SAPK3/p38g | 108 | 15 | 83 | 9 | 76 | 4 | 89 | 9 |
| SAPK4/p38d | 66 | 9 | 75 | 15 | 85 | 15 | 88 | 15 |
| MAPKAP-Kla | 77 | 15 | 55 | 12 | 60 | 14 | 58 | 8 |
| MAPKAP-K2 | 56 | 4 | 28 | 2 | 36 | 9 | 28 | 1 |
| MSK1 | 79 | 15 | 85 | 9 | 83 | 15 | 69 | 2 |
| PRAK | 48 | 5 | 9 | 4 | 9 | 5 | 9 | 5 |
| PKA | 47 | 10 | 69 | 15 | 59 | 15 | 69 | 1 |
| PKCa | 96 | 1 | 78 | 6 | 86 | 1 | 85 | 12 |
| PDK1 | 71 | 4 | 51 | 15 | 65 | 4 | 88 | 10 |
| PKB?ph | 94 | 15 | 53 | 4 | 76 | 9 | 39 | 8 |
| SGK | 14 | 15 | 3 | 5 | 7 | 4 | 4 | 3 |
| p70 S6K | 110 | 1 | 35 | 5 | 52 | 9 | 34 | 6 |
| GSK3b | 47 | 2 | 43 | 5 | 85 | 4 | 63 | 7 |
| ROCK-II | 80 | 1 | 54 | 4 | 73 | 4 | 75 | 6 |
| AMPK | 72 | 13 | 66 | 4 | 70 | 7 | 61 | 7 |
| CHK1 | 9 | 6 | 24 | 15 | 47 | 15 | 127 | 9 |
| CK2 | 80 | 0 | 79 | 3 | 85 | 2 | 80 | 0 |
| PHK | 23 | 1 | 21 | 1 | 27 | 0 | 47 | 9 |
| Lck | 76 | 4 | 60 | 12 | 54 | 1 | 71 | 13 |
| CSK | 103 | 15 | 64 | 3 | 60 | 5 | 93 | 3 |
| CDK2/cyclin A | 97 | 1 | 90 | 12 | 111 | 10 | 113 | 7 |
| DYRK1a | 21 | 3 | 27 | 6 | 16 | 5 | 25 | 10 |
| CK1 | 89 | 5 | 64 | 9 | 77 | 2 | 88 | 1 |
| NEK6 | 92 | 15 | 89 | 8 | 81 | 4 | 86 | 12 |

| | | | | | | | | |
|---|---|---|---|---|---|---|---|---|
| *Activité enzymatique résiduelle de protéine kinases en présence de composés inhibiteurs à la concentration indiquée (exprimée en pourcentage de l'activité mesurée en l'absence d'inhibiteurs). Les résultats obtenus pour le gossypol et la gossypolone sont indiqués à titre de comparaison.* | | | | | | | | |

## Revendications

1. Composés de formule générale (1) : 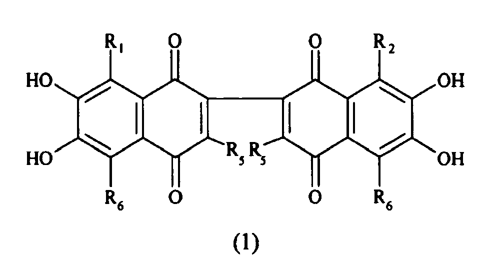 dans laquelle, indépendamment l'un de l'autre, R₁ et R₂ représentent :
- OH, ou
- CH₂-O-R₃, ou
- CH₂-S-R₃, ou 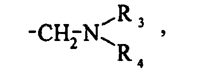
R₃ et R₄ représentent, indépendamment l'un de l'autre, -H ou un groupement carboné de 1 à 10 atomes de carbones, saturé ou non, comprenant éventuellement substitué un ou plusieurs groupements hétéroatomiques,
R₅ représente un groupement méthyle,
et R₆ représente un groupement isopropyle.

2. Composés selon la revendication 1, de formule générale (1) dans laquelle indépendamment les uns des autres, R₃ et R₄ représentent :
-H, un groupement alkyle, un groupement hétéroalkyle, tel qu'un thioalkyle, un hydroxyalkyle, un aminoalkyle, un halogénoalkyle ou un alkoxyalkyle, un groupement cycloalkyle, un groupement alcényle, un groupement hétéroalcényle, un groupement aryle, un groupement hétéroaryle, tel qu'un thioaryle, un hydroxyaryle, un aminoaryle, un halogénoaryle ou un alkoxyaryle, ou un groupement arylalkyle.

3. Composés selon l'une des revendications 1 ou 2, de formule générale (1) dans laquelle R₁ et R₂ sont identiques.

4. Composés selon l'une des revendications 1 à 3, de formule générale (2) : 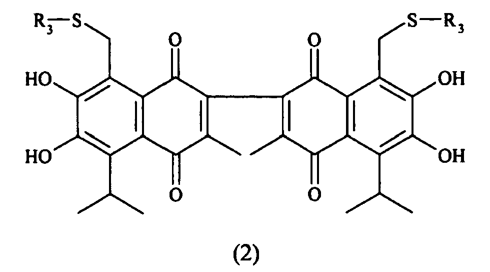 dans laquelle R₃ est tel que défini dans la revendication 1 ou 2.

5. Composés selon l'une des revendications 1 à 3, de formule générale (3) : 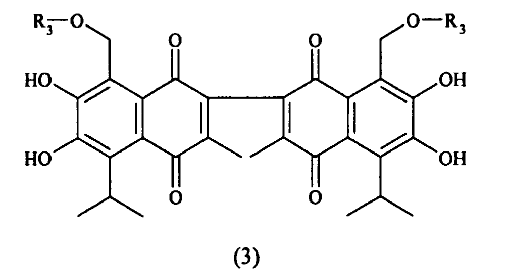 dans laquelle R₃ est tel que défini dans la revendication 1 ou 2.

6. Composés selon l'une des revendications 1 à 3, de formule suivante :

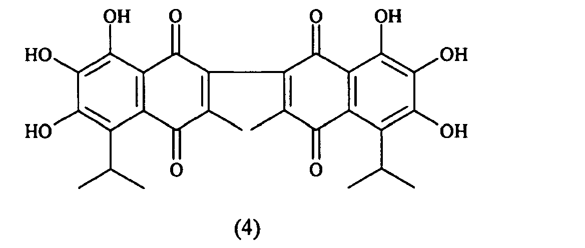

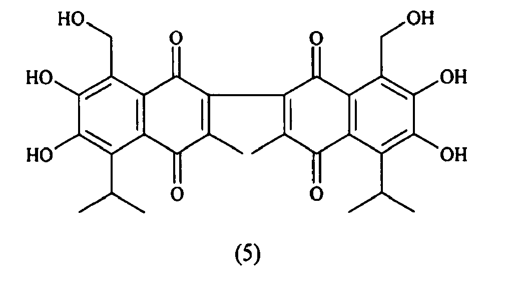

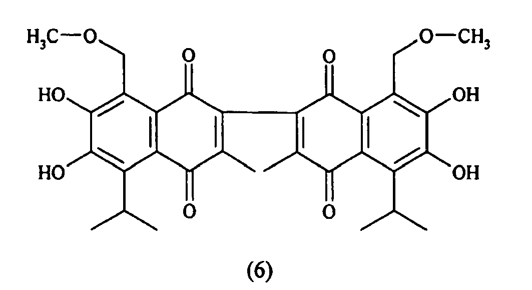

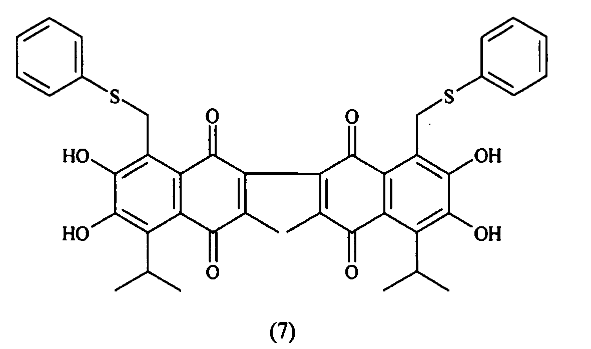

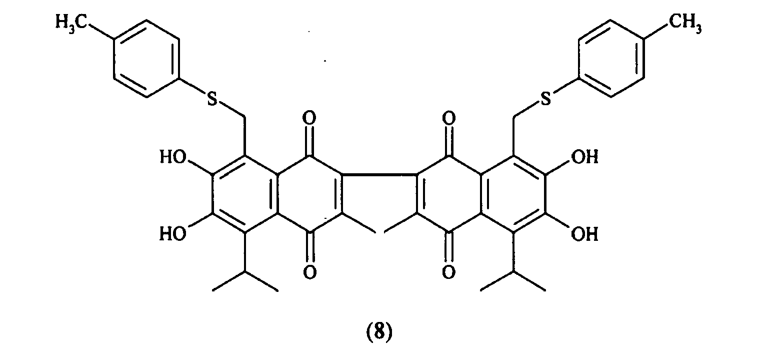

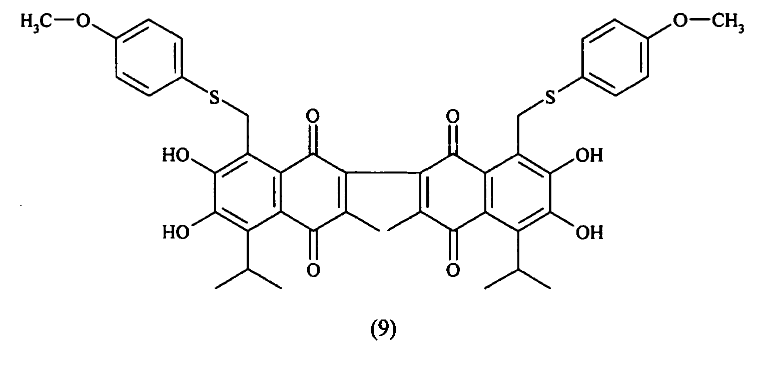

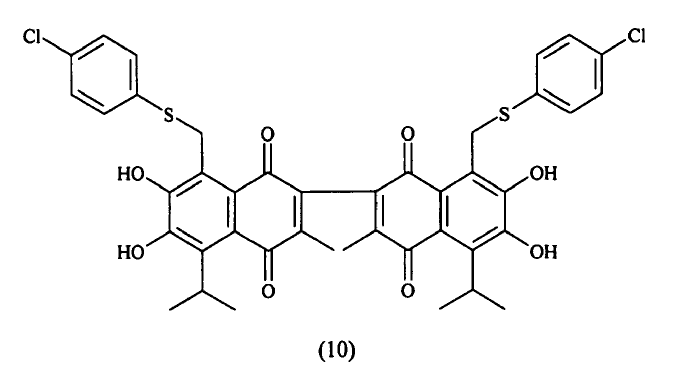

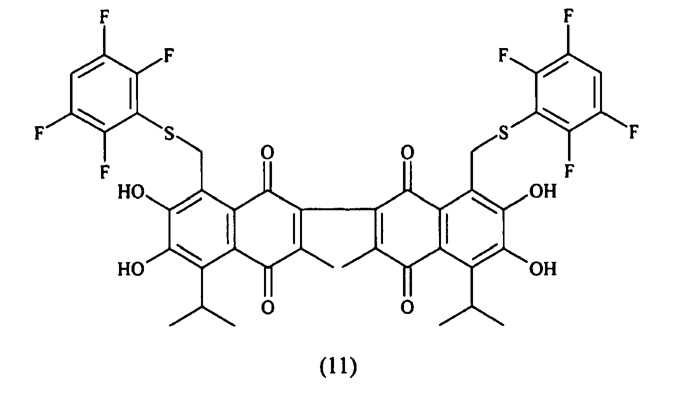

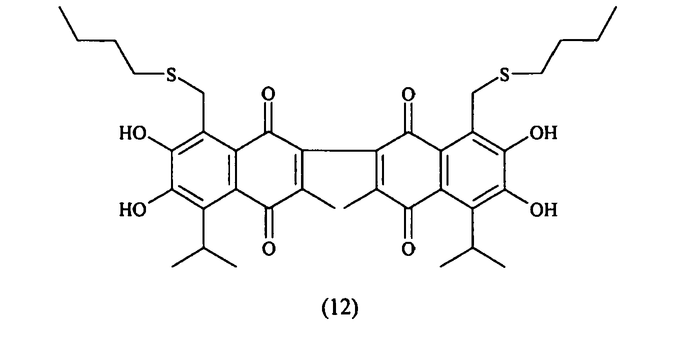

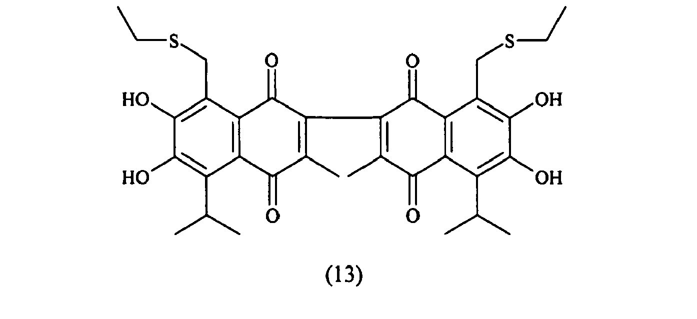

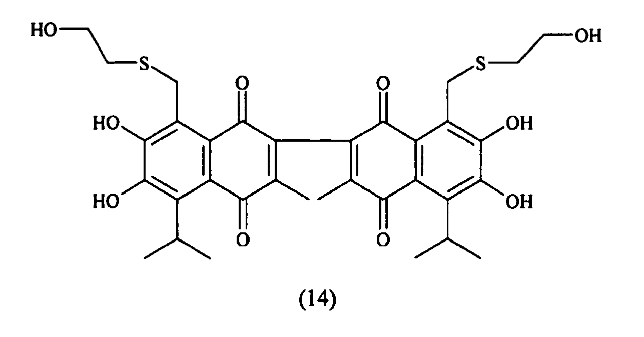

7. Composition pharmaceutique comprenant à titre de substance active au moins un composé tel que défini dans l'une des revendications précédentes, ou leurs sels pharmaceutiquement acceptables.

8. Composition pharmaceutique selon la revendication 7, comprenant à titre de substance active le composé (4) et/ou le composé (14), tels que définis dans la revendication 6.

9. Utilisation d'un composé selon l'une des revendications 1 à 6, ou de leurs sels pharmaceutiquement acceptables, pour la préparation d'un médicament destiné au traitement des cancers, des maladies parasitaires, telles que le paludisme, des viroses ou des rejets de greffe, ou à la contraception masculine.

10. Utilisation selon la revendication 9, du composé (4) et/ou du composé (14) tel que définis dans la revendication 6, pour la préparation d'un médicament destiné au traitement des cancers, tels que les mélanomes, les cancers du colon, les cancers du poumon, les glioblastomes, les adénocarcinomes, les cancers de la prostate ou les cancers du sein.

11. Procédé de préparation d'un composé de formule générale (2) tel que défini dans la revendication 4 ou d'un composé de formule (3) tel que défini dans la revendication 5, **caractérisé en ce qu'**il comprend :
- une étape de réduction de la gossypolone pour former le composé de formule (5) tel que défini dans la revendication 6, et
- une étape de substitution dudit composé de formule (5) par un composé de formule R₃-SH pour former un composé de formule (2), ou par un composé de formule R₃-OH pour former un composé de formule (3).

12. Procédé de préparation d'un composé de formule générale (4), tel que défini dans la revendication 6, **caractérisé en ce qu'**il comprend une étape d'oxydation de la gossypolone.

## Claims

1. Compounds of general formula (1): 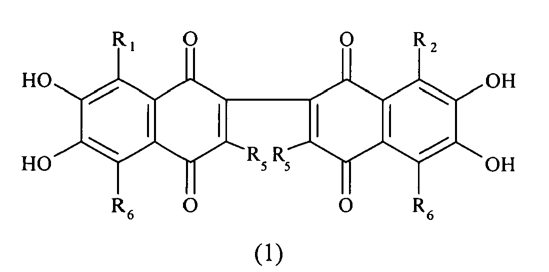 in which, independently of each other, R₁ and R₂ represent:
- OH, or
- CH₂-O-R₃, or
- CH₂-S-R₃, or 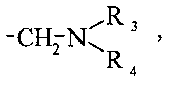
R₃, and R₄, representing, independently of each other, -H or a carbon-containing group with 1 to 10 carbon atoms, saturated or unsaturated, optionally substituted by one or more heteroatomic groups,
R₅ represents a methyl group and
R₆ represents an isopropyl group.

2. Compounds according to claim 1, of general formula (1) in which, independently of each other, R₃ and R₄, represent:
-H, an alkyl group, a heteroalkyl group, such as a thioalkyl, a hydroxyalkyl, an aminoalkyl, a halogenoalkyl or an alkoxyalkyl, a cycloalkyl group, an alkenyl group, a heteroalkenyl group, an aryl group, a heteroaryl group, such as a thioaryl, a hydroxyaryl, an aminoaryl, a halogenoaryl or an alkoxyaryl or an arylalkyl group.

3. Compounds according to one of claims 1 to 2, of general formula (1) in which R₁ and R₂ are identical.

4. Compounds according to one of claims 1 to 3, of general formula (2): 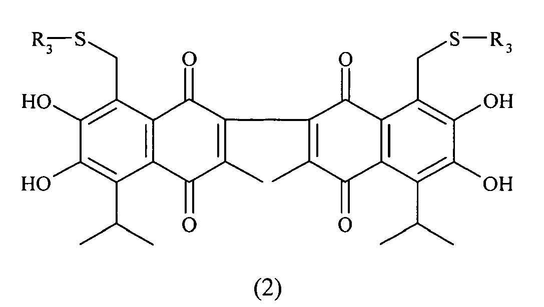 in which R₃ is as defined in claim 1 or 2.

5. Compounds according to one of claims 1 to 3, of general formula (3): 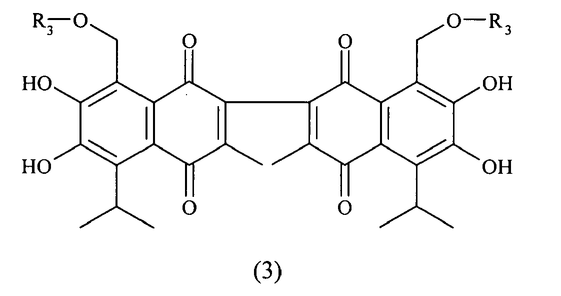 in which R₃ is as defined in claim 1 or 2.

6. Compounds according to one of claims 1 to 3, of the following formula:

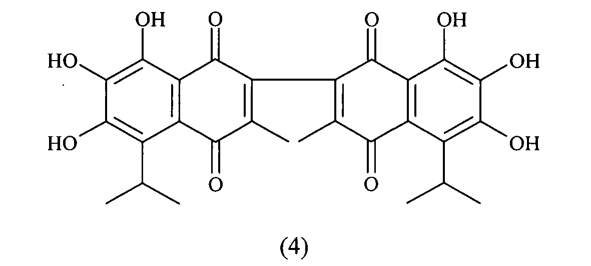

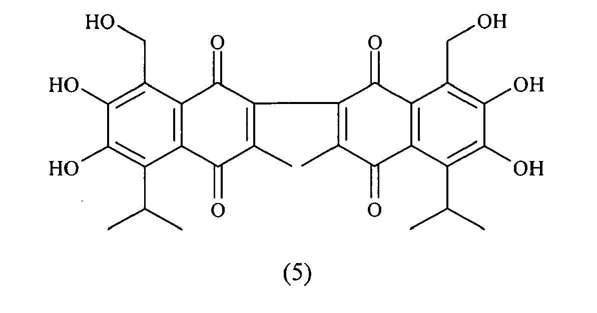

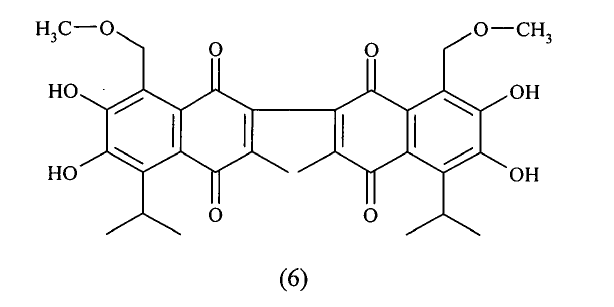

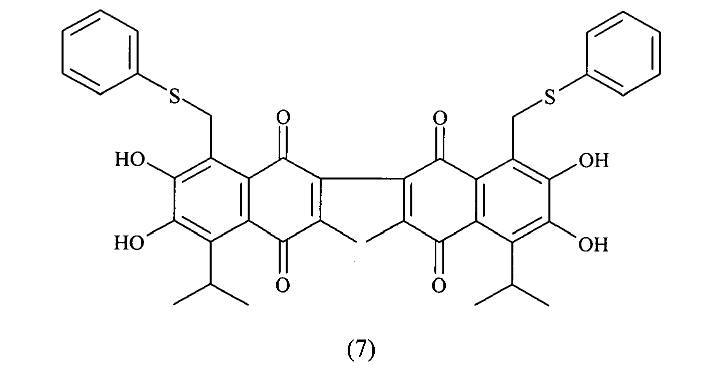

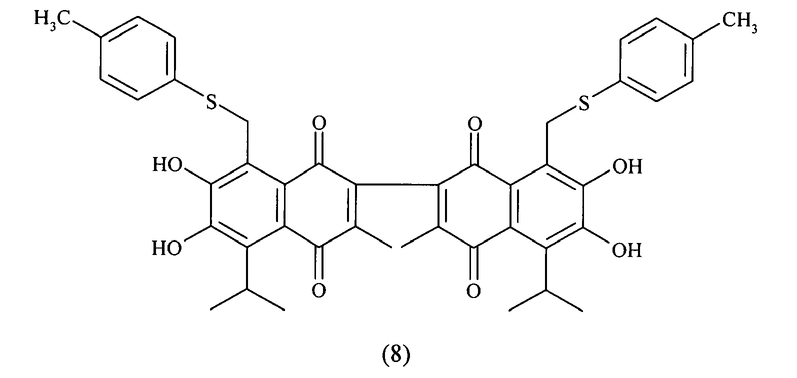

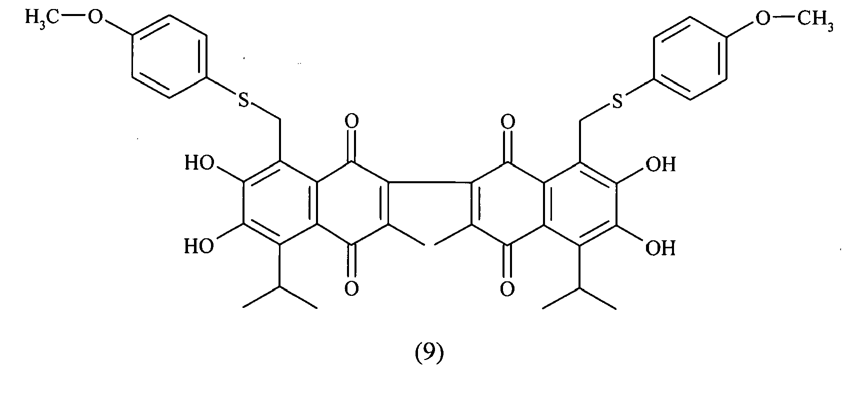

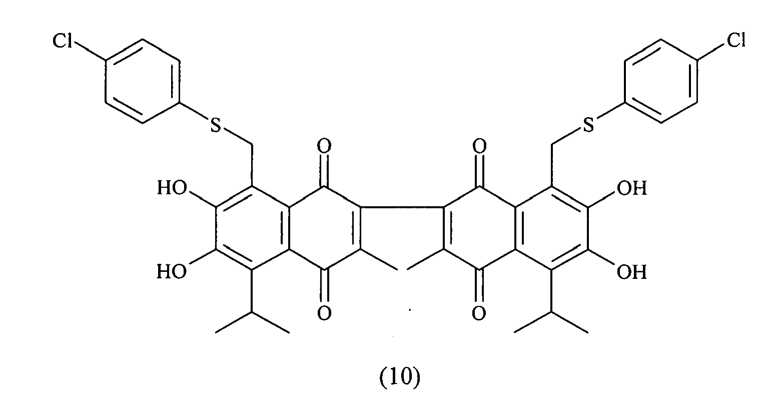

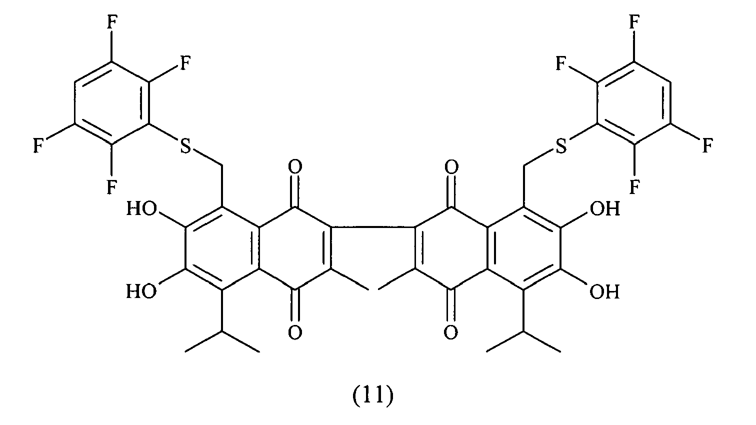

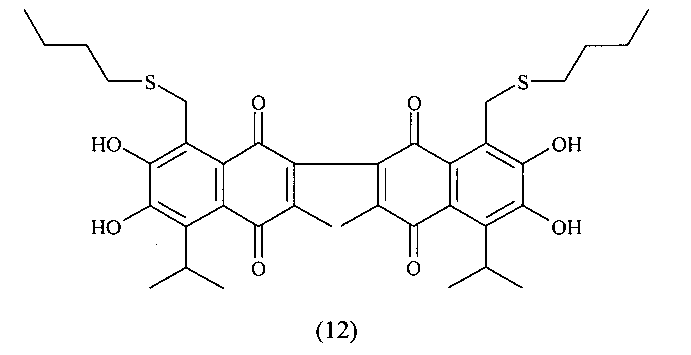

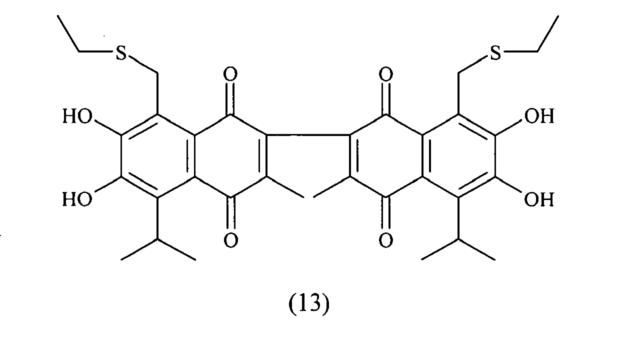

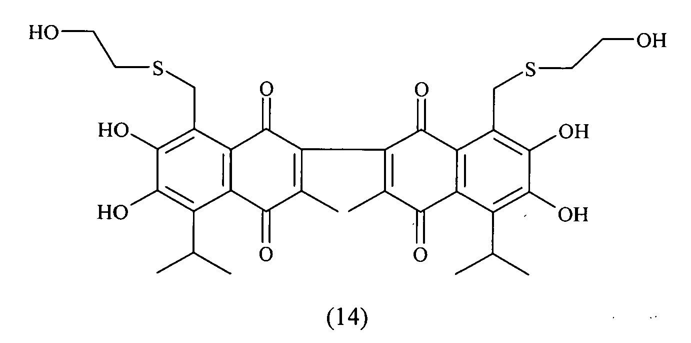

7. Pharmaceutical composition comprising as active ingredient at least one compound as defined in one of the preceding claims, or their pharmaceutically acceptable salts.

8. Pharmaceutical composition according to claim 7, comprising as active ingredient the compound (4) and/or the compound (14), as defined in claim 6.

9. Use of a compound according to one of claims 1 to 6, or their pharmaceutically acceptable salts, for the preparation of a medicament intended for the treatment of cancer, parasitoses, such as malaria, viroses or graft rejections, or for male contraception.

10. Use according to claim 9, of the compound (4) and/or of the compound (14) as defined in claim 6, for the preparation of a medicament intended for the treatment of cancers, such as melanomas, colon cancers, lung cancers, glioblastomas, adenocarcinomas, prostate cancers or breast cancers.

11. Method for the preparation of a compound of general formula (2) as defined in claim 4 or a compound of formula (3) as defined in claim 5, **characterized in that** it comprises:
- a stage of reduction of gossypolone in order to form the compound of formula (5) as defined in claim 6, and
- a stage of substitution of said compound of formula (5) with a compound of formula R₃-SH in order to form a compound of formula (2), or with a compound of formula R₃-OH in order to form a compound of formula (3).

12. Method for the preparation of a compound of general formula (4), as defined in claim 6, **characterized in that** it comprises a stage of oxidation of gossypolone.

## Patentansprüche

1. Verbindungen der allgemeinen Formel (1): 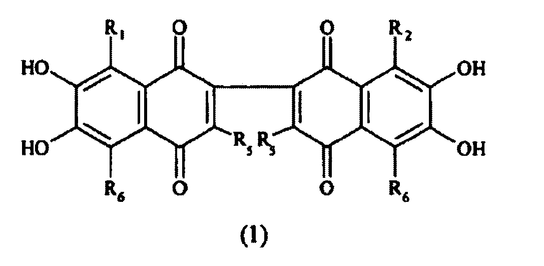 worin R₁ und R₂ unabhängig voneinander stehen für:
- OH oder
- CH₂-O-R₃, oder
- CH₂-S-R₃ oder
- 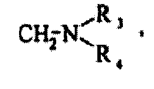
R₃ und R₄, unabhängig voneinander für -H oder eine gesättigte oder ungesättigte Kohlenstoffgruppe mit 1 bis 10 Kohlenstoffatomen stehen, die optional ein oder mehrere Heteroatomgruppen umfasst,
R₅ für eine Methylgruppe steht,
und R₆ für eine Isopropylgruppe steht.

2. Verbindungen nach Anspruch 1 mit der allgemeinen Formel (1), worin R₃ und R₄ unabhängig voneinander stehen für:
- H, eine Alkylgruppe, eine Heteroalkylgruppe, wie etwa ein Thioalkyl, ein Hydroxyalkyl, ein Aminoalkyl, ein Halogenoalkyl oder ein Alkoxyalkyl, eine Cycloalkylgruppe, eine Alkenylgruppe, eine Heteroalkenylgruppe, eine Arylgruppe, eine Heteroarylgruppe, wie etwa ein Thioaryl, ein Hydroxyaryl, ein Aminoaryl, ein Halogenoaryl oder ein Alkoxyaryl, oder eine Arylalkylgruppe.

3. Verbindungen nach einem der Ansprüche 1 oder 2 der allgemeinen Formel (1), worin R₁ und R₂ identisch sind.

4. Verbindungen nach einem der Ansprüche 1 bis 3 der allgemeinen Formel (2): 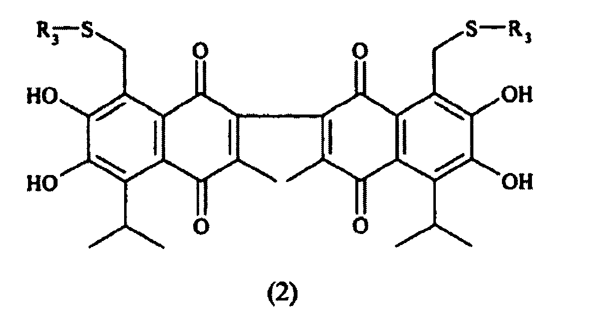 worin R₃ ist, wie in Anspruch 1 oder 2 definiert.

5. Verbindungen nach einem der Ansprüche 1 bis 3 der allgemeinen Formel (3): 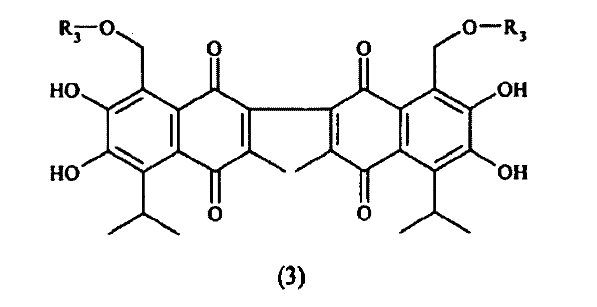 worin R₃ ist, wie in Anspruch 1 oder 2 definiert.

6. Verbindungen nach einem der Ansprüche 1 bis 3 der folgenden Formel:

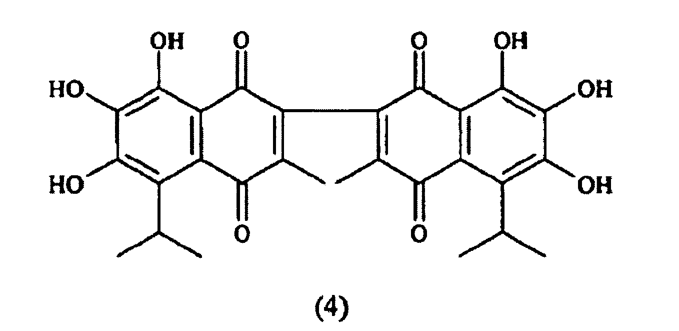

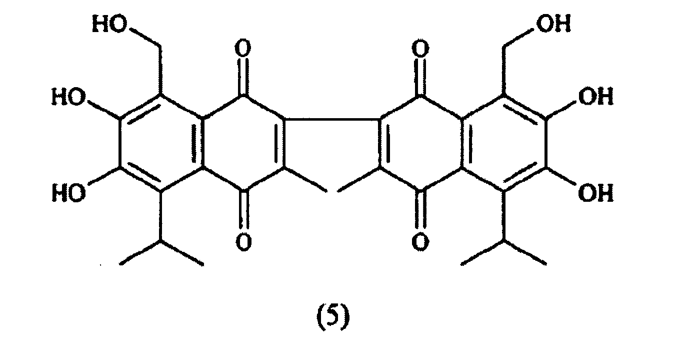

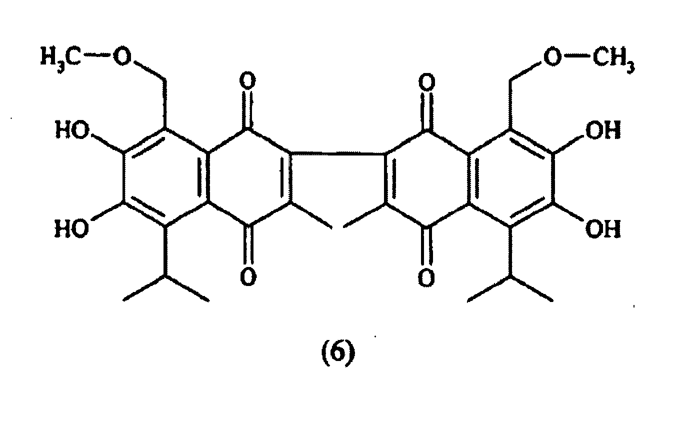

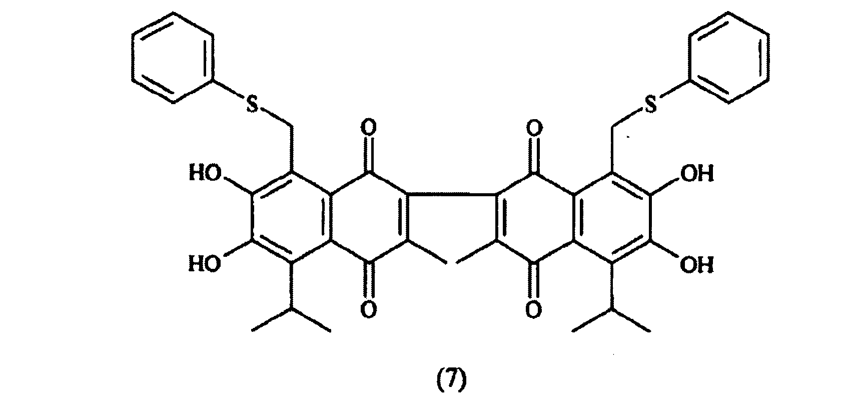

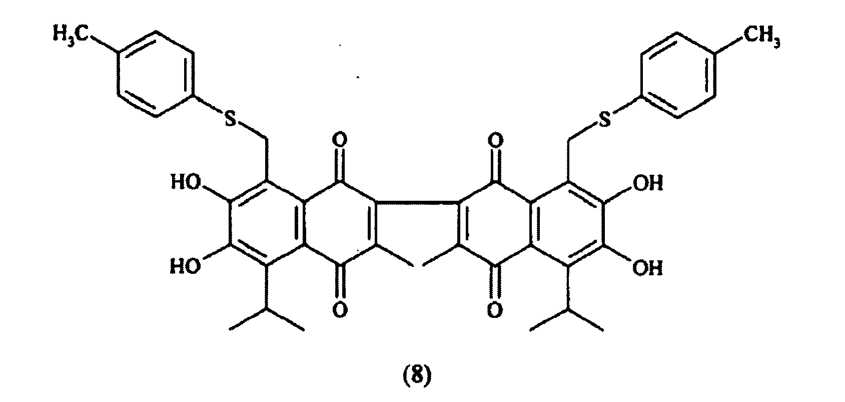

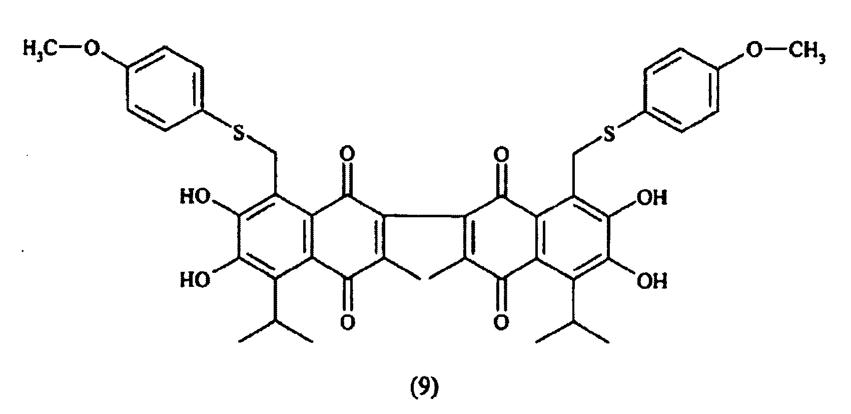

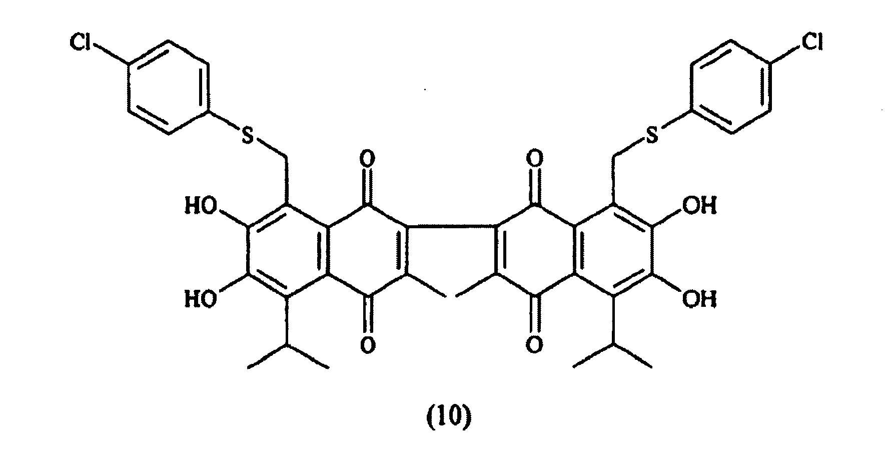

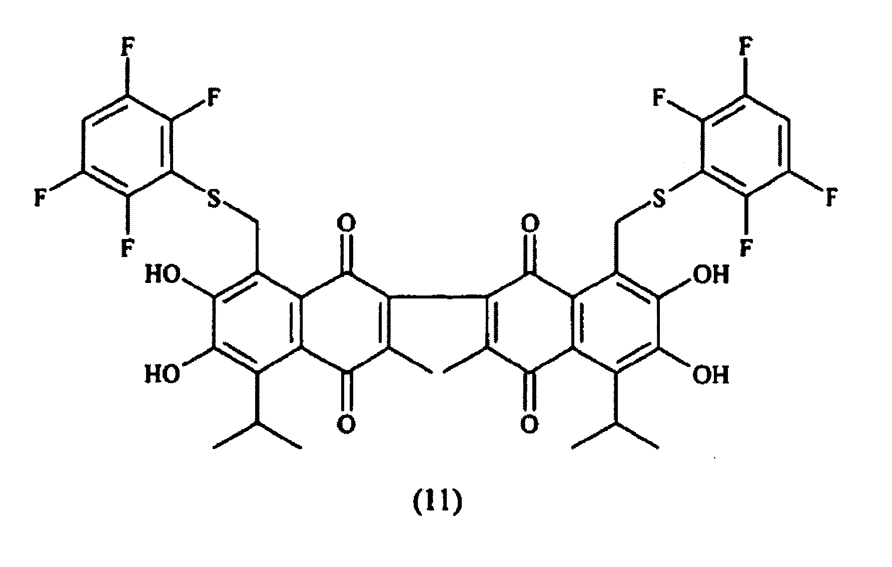

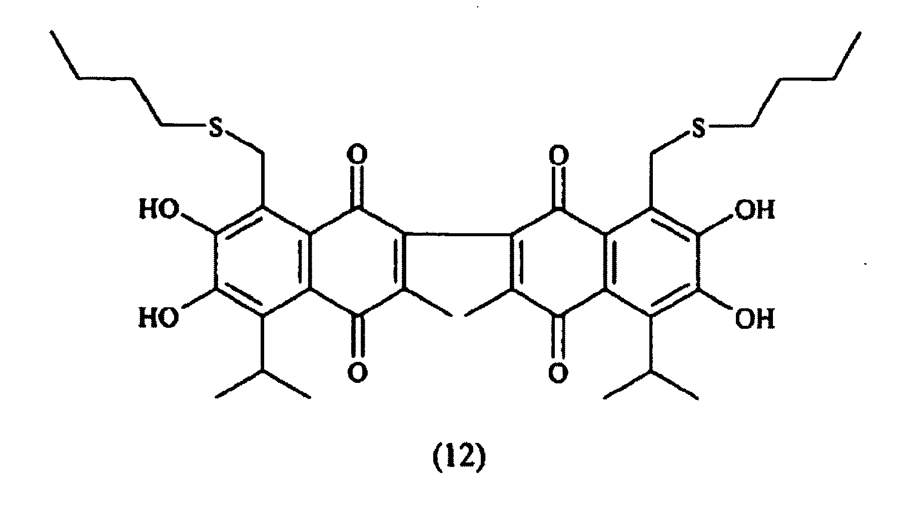

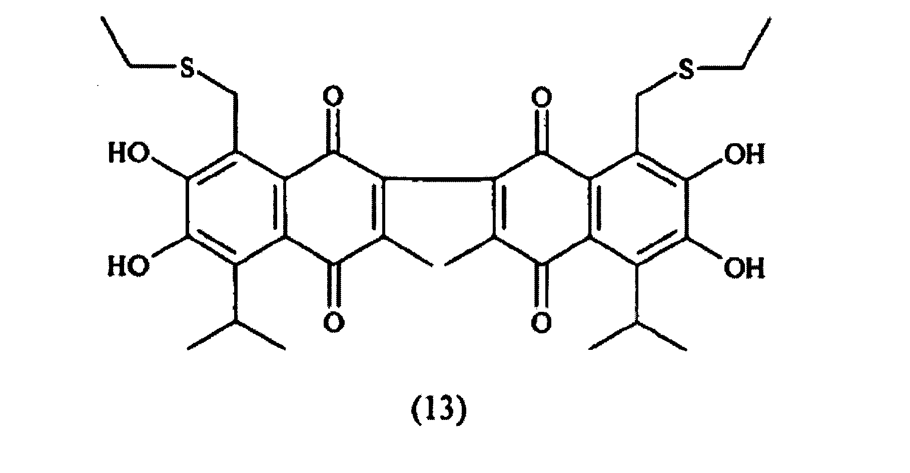

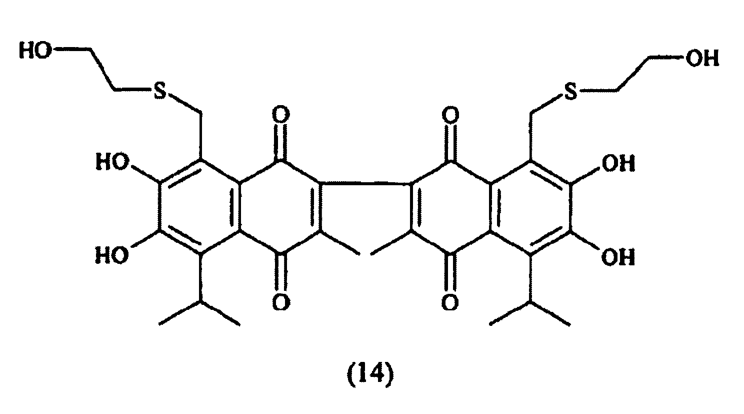

7. Pharmazeutische Zusammensetzung umfassend als Wirkstoff mindestens eine Verbindung, wie in einem der vorhergehenden Ansprüche definiert, oder deren pharmazeutisch unbedenklichen Salze.

8. Pharmazeutische Zusammensetzung nach Anspruch 7, umfassend als Wirkstoff die Verbindung (4) und/oder die Verbindung (14), wie in Anspruch 6 definiert.

9. Verwendung einer Verbindung nach einem der Ansprüche 1 bis 6, oder deren pharmazeutisch unbedenklichen Salze, zur Herstellung eines Medikaments, das zur Behandlung von Krebserkrankungen, parasitären Erkrankungen, wie etwa Malaria, Viruserkrankungen oder Transplantatabstoßungen oder zur Kontrazeption des Mannes dient.

10. Verwendung nach Anspruch 9 der Verbindung (4) und/oder der Verbindung (14), wie in Anspruch 6 definiert, zur Herstellung eines Medikaments, das der Behandlung von Krebserkrankungen, wie etwa Melanomen, Darmkrebserkrankungen, Lungenkrebserkrankungen, Glioblastomen, Adenokarzinomen, Prostata- oder Brustkrebserkrankungen dient

11. Verfahren zur Herstellung einer Verbindung der allgemeinen Formel (2), wie in Anspruch 4 definiert, oder einer Verbindung der Formel (3), wie in Anspruch 5 definiert, **dadurch gekennzeichnet, dass** es umfasst:
- einen Schritt der Reduktion von Gossypolon, um die Verbindung der Formel (5), wie in Anspruch 6 definiert, zu bilden, und
- einen Schritt der Substitution der Verbindung der Formel (5) mit einer Verbindung der Formel R₃-SH, um eine Verbindung der Formel (2) zu bilden, oder mit einer Verbindung der Formel R₃-OH, um eine Verbindung der Formel (3) zu bilden.

12. Verfahren zur Herstellung einer Verbindung der allgemeinen Formel (4), wie in Anspruch 6 definiert, **dadurch gekennzeichnet, dass** es einen Schritt der Oxidation von Gossypolon umfasst.
